# EUROPÄISCHE PATENTSCHRIFT

(11) **EP 0 392 283 B1**
(45) Veröffentlichungstag und Bekanntmachung des Hinweises auf die Patenterteilung: **15.11.1995**
(21) Anmeldenummer: 90106109.3
(22) Anmeldetag: 30.03.1990
(51) Int. Cl.: G01N 35/00, G01N 33/52

(54) **Testträger-Analysesystem**
Test-support analysis system
Système d'analyse de support de test

(30) Priorität: 08.04.1989 DE 3911539
(43) Veröffentlichungstag der Anmeldung: 17.10.1990
(73) Patentinhaber: BOEHRINGER MANNHEIM GMBH, 68298 Mannheim (DE)
(72) Erfinder: Haar, Hans-Peter, Dr.rer.nat., D-6908 Wiesloch (DE)
(74) Vertreter: Pfeifer, Hans-Peter, Dr., Dr. H.-P. Pfeifer Dr. P. Jany, Patentanwälte

(56) Entgegenhaltungen:
- EP-A- 0 073 056
- EP-A- 0 132 790
- EP-A- 0 247 439

## Beschreibung

Die Erfindung betrifft ein Testträger-Analysesystem.

Solche Systeme werden zunehmend zur Analyse von Körperflüssigkeiten, insbesondere Urin und Blut, für medizinische Zwecke eingesetzt. Die Testträger haben meist die Form von Teststreifen oder sind als flache, näherungsweise quadratische Plättchen ausgebildet. Sie enthalten eine oder mehrere Testschichten, in denen Reagenzien eingebettet sind. Diese werden mit der Probe in Kontakt gebracht, was bei der Untersuchung von Urin im allgemeinen durch Eintauchen geschieht, während zur Untersuchung von Blut üblicherweise ein Blutstropfen aufgetropft wird.

Die Reaktion der Probe mit den Reagenzien in den Testschichten führt zu einer nachweisbaren Veränderung in einer der Schichten, welche als Nachweisschicht bezeichnet wird. Meist handelt es sich um eine optisch nachweisbare Veränderung, insbesondere einen Farbumschlag. Es sind jedoch beispielsweise auch Testträger bekannt, die auf elektrochemischen Prinzipien basieren, wobei die physikalisch nachweisbare Veränderung sich auf einen elektrischen Strom oder eine elektrische Spannung bezieht.

Die physikalisch nachweisbare Veränderung wird mit Hilfe des zum System gehörenden Auswertegerätes bestimmt, das eine entsprechende Meßeinrichtung, in den genannten Fällen ein Reflexionsphotometer zur Messung des diffusen Reflexionsvermögens (Reflektivität) der Nachweisschicht bzw. eine empfindliche Strom- oder Spannungsmeßschaltung zur Erzeugung eines Meßsignals R aufweist, aus dem sich die gesuchte Konzentration C ermitteln läßt.

Die Testträger sind üblicherweise spezifisch für eine bestimmte Analyse geeignet, d.h. ein Testträgertyp dient zur Feststellung der Konzentration eines bestimmten Bestandteiles einer Körperflüssigkeit, die als Parameter bezeichnet wird. Für einen bestimmten Testträgertyp, beispielsweise zur Analyse von Glucose oder von Cholesterin in Blut, besteht ein bestimmter Zusammenhang zwischen dem Meßsignal R und der Konzentration C. Dieser wird als Auswertekurve bezeichnet.

Hierbei besteht allerdings ein Problem, welches bereits seit den Anfängen der quantitativen Analytik mit Hilfe von Testträgern bekannt ist, nämlich, daß die Testträger chargenweise produziert werden, und es in aller Regel nicht möglich ist, den Herstellungsvorgang so exakt reproduzierbar zu gestalten, daß auch bei hohen Anforderungen an die Genauigkeit der Analyse die gleiche Auswertekurve für verschiedene Herstellungschargen verwendet werden kann. Es hat nicht an Versuchen gefehlt, dieses Problem zu lösen.

Die einfachste Lösung ist der Verzicht auf eine chargenspezifische Korrektur. Auch heute noch werden Testträger-Analysesysteme angeboten, bei denen dem Auswertegerät eine mittlere Auswertekurve einprogrammiert ist, wobei die damit zwangsläufig verbundenen Ungenauigkeiten in Kauf genommen werden. Dabei muß man bedenken, daß in den meisten Fällen aufgrund physikalisch-chemischer Gesetzmäßigkeiten der Signalhub, d.h. die Gesamtänderung der physikalischen Meßgröße im medizinisch relevanten Meßbereich verhältnismäßig gering ist. Dies hat zur Folge, daß schon verhältnismäßig kleine Fehler in der Auswertekurve zu großen Abweichungen bei der gemessenen Konzentration C führen.

Ein zweiter Weg ist die Eichung mit Hilfe von Kalibrationsflüssigkeiten bekannter Konzentration. Bei Systemen, die nach diesem Prinzip arbeiten, ist in dem Auswertegerät ebenfalls eine mittlere Auswertekurve abgespeichert, die jedoch von Fall zu Fall aufgrund von Kalibrationsmessungen korrigiert werden kann. Dieses Verfahren ist jedoch zeitraubend und kompliziert, und zwar um so mehr, je höher die angestrebte Genauigkeit ist. Im Regelfall ist es nämlich nicht ausreichend, nur mit einer einzigen Konzentration zu kalibrieren, wenn eine gute Genauigkeit über den gesamten Meßbereich angestrebt wird. Vor allem bei Analysesystemen, die zur Anwendung durch Laien (sogenanntes "home-monitoring") vorgesehen sind, ist dieser Weg deswegen wenig geeignet.

Um diese Probleme zu vermeiden, wurden mehrere Lösungen vorgeschlagen, bei denen die zur Berücksichtigung der chargenspezifischen Auswertekurve notwendige Information an das Auswertegerät übermittelt und von diesem bei der Umwandlung des Meßsignals R in die Konzentration C berücksichtigt wird.

Bereits eines der ersten Systeme zur Analyse von Glucose, welches noch analogelektronisch arbeitete, berücksichtigte die chargenspezifische Auswertekurve durch auswechselbare Skalenscheiben, die den jeweiligen Teststreifenpackungen beigegeben waren. In der europäischen Patentanmeldung EP-A 0073056 (entsprechend USA 4 592 893) ist ein System beschrieben, dessen Testträger einen Strichcode aufweisen, der die chargenspezifische Auswertekurve enthält. Das zugehörige Auswertegerät enthält einen Strichcode-Leser zur Erfassung der Information. Die EP-A 132790 (entsprechend USA 4 578 716) befaßt sich mit der Speicherung der Information in einer auf den Testträgern befindlichen Magnetschicht. Das Auswertegerät hat einen entsprechenden Magnetlesekopf. Bei dem in der EP-A 247 439 beschriebenen System enthält das Gerät gewisse Grundinformationen über die chargenspezifische Auswertekurve, nämlich eine Standardauswertekurve und Berechnungsstützpunkte. Dadurch ist das Gerät in der Lage, eine chargenspezifische Auswertekurve, die der wahren Auswertekurve sehr nahe kommt, zu generieren, wenn ihm eine dreistellige Zahl eingegeben wird, die zur Auswahl der jeweils zu verwendenden Berechnungsstützpunkte führt.

Der Erfindung liegt die Aufgabe zugrunde, ein Testträger-Analysesystem zur Verfügung zu stellen, welches ähnlich einfach und damit kostengünstig ist, wie Systeme ohne chargenspezifische Korrektur und dennoch diese Korrektur ermöglicht. Dabei soll die Handhabung einfach und sicher sein.

Das erfindungsgemäße Testträger-Analysesystem, welches diese Aufgabe löst, umfaßt
- Testträger, welche in einer oder mehreren Testschichten Reagenzien enthalten, wobei die Reaktion der Reagenzien mit dem Bestandteil zu einer physikalisch-nachweisbaren Veränderung einer Nachweisschicht führt, die Veränderung in einem bestimmten, einer Auswertekurve folgenden Zusammenhang zu der Konzentration des Bestandteils steht und die Auswertekurve von der Herstellungscharge des Testträgers abhängig ist,
- ein Auswertegerät, das
   - eine Meß- und Digitalisierungsschaltung zur Messung der physikalisch nachweisbaren Veränderung, Erzeugung eines Meßsignals R und Umwandlung des Meßsignals R in ein von der Auswertekurve unabhängiges Zwischenergebnis durch Digitalisierung des Meßsignals R in eine endliche Zahl von diskreten Ausgangszuständen Xᵢ und
   - ein Display zur Anzeige der Ausgangszustände in Form eines alphanumerischen Codes
   enthält, und
- ein lesbares Informationsträgerelement mit einer Zuordnung zwischen den den diskreten Ausgangszuständen Xᵢ entsprechenden alphanumerischen Codes des Display und unter Berücksichtigung der Auswertekurve ermittelten Endergebniswerten. Die Endergebniswerte sind vorzugsweise Konzentrationswerte des zu bestimmenden Bestandteils. Es können jedoch auch andere aus dem Analyseergebnis abgeleitete Informationen sein.

Die mit der Erfindung erzielten Vorteile beziehen sich auf jedes der drei Elemente des Systems:
- Bei der Herstellung der Testträger muß kein besonderer Aufwand getrieben werden, um die verschiedenen Produktionschargen möglichst gleich herzustellen. Es ist nicht einmal erforderlich, daß die Auswertekurve jeweils den gleichen allgemeinen Verlauf hat.
- Das Auswertegerät kann extrem einfach aufgebaut sein. Es enthält - abgesehen von selbstverständlichen Elementen wie einer Spannungsversorgung und der Einheit zur Erfassung der physikalisch nachweisbaren Veränderung (beispielsweise Meßoptik) - in der einfachsten Version lediglich einen Meßverstärker und eine Digitalisierungsschaltung. Insbesondere ist kein Mikroprozessor mit zugehörigen Speicherbausteinen erforderlich.
- Trotz dieses einfachen Aufbaus ist ein einziges Auswertegerät nicht nur zur Auswertung eines Testträgers geeignet, sondern es können verschiedene Testträger für verschiedene Parameter mit dem gleichen Gerät ausgewertet werden. Es ist nicht einmal erforderlich, daß zum Zeitpunkt der Herstellung des Gerätes die spezifischen Eigenschaften künftiger Testträger im einzelnen festliegen, solange sie nur hinsichtlich des Meßprinzips, also der physikalisch nachweisbaren Veränderung und selbstverständlich hinsichtlich des äußeren Aufbaus an das Auswertegerät angepaßt sind.
- Als Informationsträgerelement kann einfach die Verpackung der Testträger verwendet werden. Es kann jedoch auch zweckmäßig sein, den Testträgern eine spezielle Auswertekarte beizufügen oder die Zuordnung zwischen den Codes des Display und den Konzentrationswerten auf den Testträgern selbst (beispielsweise auf der Rückseite) vorzusehen, um damit jede Verwechslungsgefahr auszuschließen.

Die Erfindung wird im folgenden anhand eines in den Figuren schematisch dargestellten Ausführungsbeispiels näher erläutert; es zeigen:
- Fig. 1: ein erfindungsgemäßes Testträger-Analysesystem in perspektivischer Darstellung,
- Fig. 2: ein abgewandeltes Informationsträgerelement,
- Fig. 3: ein Blockschaltbild eines Auswertegerätes für ein erfindungsgemäßes System.
- Fig. 4: eine graphische Darstellung zur Erläuterung einer bevorzugten Ausführungsform.

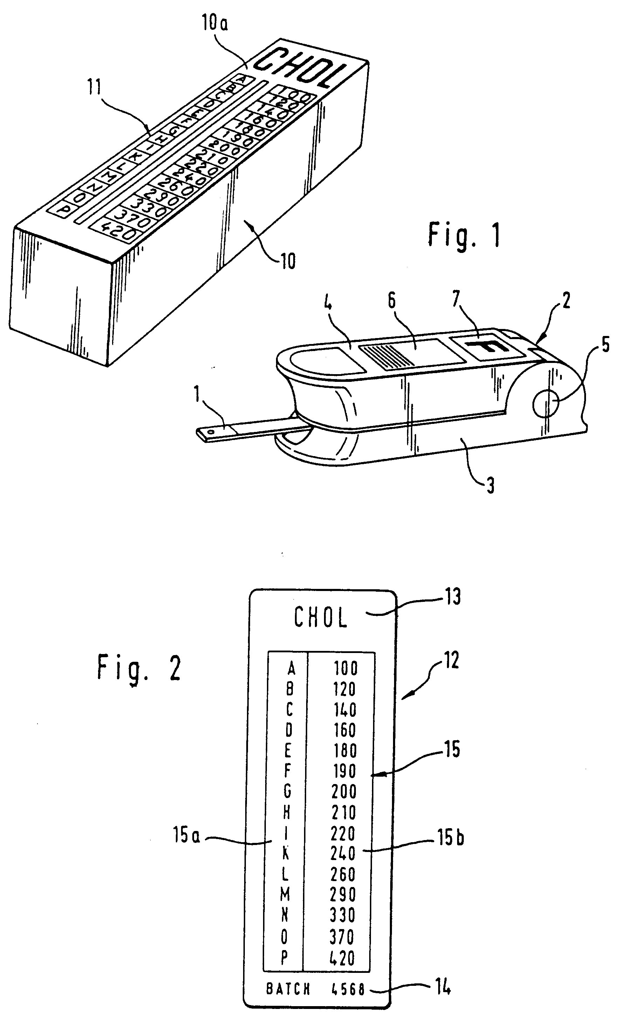

Der in Figur 1 dargestellte Testträger 1 ist in ein Auswertegerät 2 eingelegt. Im dargestellten Fall besteht das Auswertegerät 2 aus einem Unterteil 3 und einem Oberteil 4, die durch ein Scharnier 5 miteinander verbunden sind.

Zur Aufnahme des Testträgers 1 läßt sich das Oberteil 4 hochklappen. Diese besonders kleine und zweckmäßige Bauweise, bei der das gesamte Gerät als Halterung für den Testträger dient, wird dadurch ermöglicht, daß die Auswerteelektronik erfindungsgemäß extrem klein miniaturisiert werden kann. Insgesamt wird dadurch die Baugröße des Gerätes überwiegend durch die zur Aufnahme des Testträgers notwendigen mechanischen Maßnahmen bestimmt.

Auf der Oberseite des Gerätes ist neben einem Batteriefach 6 ein Display 7 angeordnet, das einen alphanumerischen Code (in der Figur den Buchstaben F) anzeigt. Dieser Code ist visuell lesbar und hat eine verhältnismäßig geringe Zahl diskreter Zustände zur Anzeige eines von der Auswertekurve unabhängigen Zwischenergebnisses. Als Code geeignet sind beispielsweise die Buchstaben des Alphabets.

Die Zahl der Zustände der Displayanzeige sollte generell relativ niedrig sein. Bevorzugt sind maximal 40, besonders bevorzugt 10 bis 30 Zustände. Statt der dargestellten Buchstabenanzeige kann auch eine andere leicht lesbare Anzeige vorgesehen sein, insbesondere eine zweistellige Zahl.

Als Informationsträgerelement dient eine Testträgerpackung 10. Sie enthält die Zuordnung zwischen den alphanumerischen Codes des Display 7 und den zugehörigen Konzentrationswerten in Form einer Tabelle 11, die im dargestellten Fall auf eine Seitenfläche 10a der Testträgerpackung 10 aufgedruckt ist.

Figur 2 zeigt als weiteres Beispiel eines geeigneten Informationsträgerelementes eine Auswertekarte 12. Das Informationsträgerelement wird vom Hersteller der Testträger jeweils zur Auswertung einer bestimmten Charge eines bestimmten Testträgertyps geliefert. Entsprechend enthält es eine Angabe über den zu bestimmenden Parameter (in diesem Fall den Code CHOL für Cholesterin, Parameterfeld 13) sowie die Chargennummer (Chargenfeld 14).

Die Zuordnungstabelle 15 berücksichtigt die chargenspezifische Auswertekurve. Demzufolge ist der Konzentrationswert in der Konzentrationsspalte 15b, der einem bestimmten alphanumerischen Code in der Codespalte 15a zugeordnet ist, von der jeweiligen Herstellungscharge abhängig. Während bei einer Charge, beispielsweise - wie dargestellt - dem Code F eine Konzentration von 190mmol/l zugeordnet ist, kann bei einer anderen Charge dem gleichen Code ein Konzentrationswert von 180 oder 200mmol/l zugeordnet sein. Die Erfindung richtet sich nicht auf irgend eine bestimmte Zuordnung oder ein zu deren Ermittlung zweckmäßiges mathematisches Verfahren, sondern auf die in den Ansprüchen definierte Problemlösung, die zu einem Testträger-Auswertesystem mit einfachstem Aufbau bei gleichzeitiger universeller Anwendbarkeit und hoher Genauigkeit führt.

Statt der Konzentrationswerte kann die Zuordnungstabelle das Analyseergebnis auch in anderer Form anzeigen, beispielsweise durch eine Bereichseinteilung ("erniedrigt - normal - erhöht - stark erhöht").

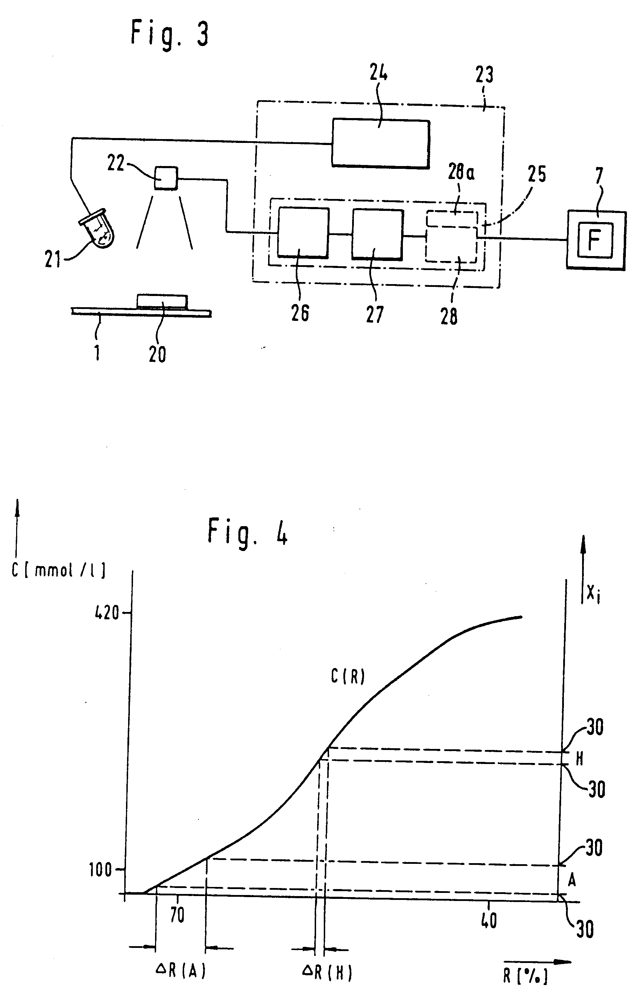

Figur 3 zeigt die prinzipiellen Elemente eines für die Erfindung geeigneten Auswertegerätes in schematischer Darstellung bzw. als Blockschaltbild.

Im dargestellten Fall ist die diffuse Reflexion der Nachweisschicht 20 eines Testträgers 1 zu bestimmen. Sie wird von einer als Lichtsender 21 dienenden Leuchtdiode beleuchtet. Das diffus reflektierte Licht wird von einem Lichtempfänger 22, beispielsweise einem Phototransistor, erfaßt.

Eine insgesamt mit 23 bezeichnete Elektronikeinheit enthält eine Lichtsenderansteuerung 24 und eine Meß- und Digitalisierungsschaltung 25, die einen Meßverstärker 26, eine Digitalisierungsschaltung 27 und eine optionale und deshalb gestrichelt gezeichnete Signalaufbereitungsschaltung 28 umfaßt. Das Ausgangssignal der Meß- und Digitalisierungsschaltung 25 liegt an dem Display 7 an.

Das von dem Lichtempfänger 22 erzeugte Meßsignal wird im einfachsten Fall lediglich von dem Verstärker 26 verstärkt, von der Digitalisierungsschaltung 27 in einen Ausgangszustand der Meß- und-Digitalisierungsschailtung 25 digitalisiert, wobei deren Zahl verhältnismäßig klein ist. Der Ausgangszustand wird als Zwischenergebnis auf dem Display 7 angezeigt. Die Zuordnung zwischen den Ausgangszuständen und den Konzentrationswerten ist auf dem visuell lesbaren Codeträgerelement enthalten. Das Auswertegerät selbst benötigt deswegen weder die Information über die chargenspezivische Auswertekurve noch aufwendige elektronische Bauteile, um aus den Meßsignalen die Konzentrationswerte unter Berücksichtigung dieser Auswertekurve zu ermitteln.

Damit soll jedoch nicht ausgeschlossen werden, daß die Elektronikeinheit 23 andere Signalverarbeitungskomponenten enthält, die in Fig. 3 durch die Signalverarbeitungsschaltung 28 symbolisiert sind.

So kann es insbesondere zweckmäßig sein, als Digitalisierungsschaltung ein kommerziell für diesen Zweck erhältliches Bauelement zu verwenden, durch das das Meßsignal in eine Vielzahl von Zuständen (beispielsweise 256 Zustände) digitalisiert wird. Die Signalaufbereitungsschaltung enthält in einem solchen Fall logische Bauelemente, die die Gesamtzahl der Zustände am Ausgang der Digitalisierungsschaltung in eine Anzahl von Teilbereichen unterteilen, die jeweils einem Zustand des alphanumerischen Codes entsprechen.

Allgemein ausgedrückt setzt die Meß- und Digitalisierungsschaltung 25 Diskriminatorschwellen, durch die die Zustände des alphanumerischen Codes dem Wertebereich des Meßsignals zugeordnet werden. Dieser Zusammenhang muß nicht linear sein, d.h. ein Ausgangszustand der Meß- und Digitalisierungsschaltung 25 muß nicht einer bestimmten Signaldifferenz des Meßsignals entsprechen. Vielmehr ist es häufig zweckmäßig, bewußt einen nichtlinearen Zusammenhang zu wählen.

Beispielsweise haben die Meßsignalkurven in der Praxis häufig einen erheblich gekrümmten Verlauf, und es kann zweckmäßig sein, sie zu linearisieren. Es kann auch vorteilhaft sein, den Signalkurvenverlauf in dem unter medizinischen Aspekten interessanten Bereich mit elektronischen Mitteln zu spreizen, um damit eine bessere Auflösung der Konzentrationswerte in diesem Bereich zu erzielen. Dies läßt sich einfach dadurch erreichen, daß die Diskriminatorschwellen für die in diesem Bereich liegenden Ausgangszustände enger beieinander liegen, als bei den medizinisch weniger interessanten Bereichen.

Diese bevorzugten Maßnahmen lassen sich anhand von Figur 4 erläutern. Dargestellt ist eine Auswertekurve C (R), d.h. die Abhängigkeit einer Konzentration (hier ausgedrückt in mmol/l) von dem Meßwert R (hier die Reflektivität einer Testschicht bei einer bestimmten Wellenlänge, ausgedrückt in %). Man erkennt, daß dem gesamten medizinisch interessanten Wertebereich von 100 bis 420 mmol/l nur ein verhältnismäßig geringer Remissionshub von 70 bis 40 % gegenübersteht. Außerdem ist nachteilig, daß im interessantesten mittleren Konzentrationsbereich die Auswertekurve verhältnismäßig steil verläuft, d.h. einer großen Änderung von C steht nur eine verhältnismäßig kleine Änderung von R gegenüber.

In einem solchen Fall wird die Genauigkeit der Auswertung verbessert, wenn die Diskriminatorschwellen 30, von denen einige beispielhaft in der Figur auf der rechten Ordinate eingezeichnet sind, im mittleren Wertebereich verhältnismäßig eng und in den Randbereichen weit gesetzt werden. Mit anderen Worten wird in dem medizinisch interessantesten Bereich einer verhältnismäßig kleinen Meßsignaldifferenz Δ R ein Ausgangszustand (im dargestellten Fall der Zustand H) zugeordnet, während in den Randbereichen einem Ausgangszustand (hier A) ein verhältnismäßig breiter Teil Δ R (A) des gesamten Meßbereiches entspricht.

Die Erfindung erlaubt auch eine besonders einfache Korrektur des Temperatureinflusses. Zu diesem Zweck ist ein Temperaturmeßaufnehmer 28a vorgesehen, dessen Meßergebnis die Signalaufbereitung beeinflußt. Dies kann am einfachsten in der Weise geschehen, daß die Ausgangszustände der Meß- und Digitalisierungsschaltung jeweils auf den nächsten benachbarten Zustand erhöht bzw. erniedrigt werden, wenn sich die Temperatur um einen bestimmten Betrag (z.B. 3°C oder 5°C) ändert.

Die Mittel zur technischen Realisierung dieser bevorzugten Maßnahmen sind dem Fachmann geläufig. Die Signalaufbereitung geschieht bevorzugt in digitaler Form, beispielsweise wie oben erläutert mit Hilfe einer konventionellen Digitalisierungsschaltung und einer nachfolgenden Logikeinheit. In diesem Fall befindet sich die Signalaufbereitungsschaltung - wie dargestellt - im Signalweg hinter der Digitalisierungsschaltung.

Sofern die Signalaufbereitung mit analogelektronischen Mitteln stattfinden soll, kann es jedoch auch zweckmäßig sein, sie im Signalweg zwischen Meßverstärker und Digitalisierungsschaltung vorzusehen. Schließlich ist auch eine spezielle Digitalisierungsschaltung möglich, die die Funktion der Signalaufbereitung mit umfaßt.

Wie erwähnt, erhält das Auswertegerät bei der vorliegenden Erfindung keinerlei Informationen über die chargenspezifische Auswertekurve. Es ist demzufolge auch nicht möglich, die Diskriminatorschwellen für die Ausgangszustände der Meß- und Auswerteschaltung jeweils so zu wählen, daß ein alphanumerischer Code jeweils einem runden Konzentrationswert entspricht. In der Praxis ist es dennoch in aller Regel ausreichend, auf dem Informationsträgerelement jeweils den nächstliegenden runden Konzentrationswert dem jeweiligen Code zuzuordnen, wie dies in Fig. 1 und 2 dargestellt ist.

Die Signalaufbereitungsschaltung kann bei einem für die Auswertung mehrerer Parameter geeigneten Auswertegerät auch dazu dienen, die verschiedenen Kurvenverläufe des Meßsignals im jeweiligen Meßbereich einander anzugleichen, um jeweils die möglichen Zustände des Display gleichmäßig auf den gesamten Meßbereich zu verteilen und damit eine möglichst genaue Auswertung zu ermöglichen. Wenn beispielsweise der Signalverlauf bei einem Testträger für Glucose verhältnismäßig steil ist, während ein Cholesterin-Testträger nur einen geringen Signalhub hat, ist es zweckmäßig, mit Hilfe der Signalaufbereitungsschaltung das Cholesterinsignal so weit zu spreizen, daß sein Gesamtbereich dem des Glucosesignals entspricht. Zu diesem Zweck muß das Auswertegerät selbstverständlich entsprechende Informationen über den auszuwertenden Testträgertyp und über den allgemeinen Verlauf der jeweiligen Auswertekurve übermittelt bekommen bzw. enthalten. Dennoch ist auch bei einer solchen Ausführungsform der Gesamtaufwand noch gering im Verhältnis zu einem bisher bekannten Mehrparametergerät.

## Patentansprüche

1. Testträger-Analysesystem zur Analyse eines Bestandteils einer flüssigen Probe, insbesondere einer Körperflüssigkeit, umfassend
a) Testträger (1), welche in einer oder mehreren Testschichten Reagenzien enthalten, wobei die Reaktion der Reagenzien mit dem Bestandteil zu einer physikalisch nachweisbaren Veränderung einer NachweisSchicht (20) führt, die Veränderung in einem bestimmten, einer Auswertekurve folgenden Zusammenhang zu der Konzentration des Bestandteils steht und die Auswertekurve von der Herstellungscharge des Testträgers abhängig ist,
b) ein Auswertegerät (2), das
- eine Meß- und Digitalisierungsschaltung (25) zur Messung der physikalisch nachweisbaren Veränderung, Erzeugung eines Meßsignals (R) und Umwandlung des Meßsignals (R) in ein von der Auswertekurve unabhängiges Zwischenergebnis durch Digitalisierung des Meßsignals (R) in eine endliche Zahl von diskreten Ausgangszuständen (Xᵢ) und
- ein Display (7) zur Anzeige der Ausgangszustände in Form eines alphanumerischen Codes
enthält, und
c) ein lesbares Informationstragerelement (10a,12) mit einer Zuordnung zwischen den den diskreten Ausgangszuständen (Xᵢ) entsprechenden alphanumerischen Codes des Display (7) und unter Berücksichtigung der Auswertekurve ermittelten Endergebniswerten.

2. Testträger-Analysesystem nach Anspruch 1, **dadurch gekennzeichnet**, daß die Zahl der Ausgangszustände der Meß- und Digitalisierungsschaltung (25) kleiner als 40 ist.

3. Testträger-Analysesystem nach Anspruch 2, **dadurch gekennzeichnet**, daß die Zahl der Ausgangszustände der Meß- und Digitalisierungsschaltung (25) zwischen 10 und 30 liegt.

4. Testträger-Analysesystem nach Anspruch 1, **dadurch gekennzeichnet**, daß die Meß- und Digitalisierungsschaltung (25) so ausgebildet ist, daß in verschiedenen Teilen des Wertebereichs des Meßsignals (R) einem Ausgangszustand (Xᵢ) der Meß- und Digitalisierungsschaltung unterschiedliche Meßsignaldifferenzen (Δ R (Xᵢ)) zugeordnet sind.

5. Testträger-Analysesystem nach Anspruch 1, **dadurch gekennzeichnet**, daß zur temperaturabhängigen Korrektur des Zwischenergebnisses ein Temperaturmeßaufnehmer (28a) vorgesehen ist, der mit der Meß- und Digitalisierungschaltung (25) verbunden ist.

## Claims

1. Test carrier analysis system for analysing a component of a fluid sample, in particular of a body fluid, comprising
a) test carriers (1) containing in one or more test layers reagents, in which the reaction of the reagents with the component leads to a physically detectable change in a detection layer (20), the change follows a particular relationship, obeying an evaluation curve, to the concentration of the component, and the evaluation curve is dependent on the manufacturing batch of the test carrier,
b) an evaluation instrument (2) containing
- a measurement and digitization circuit (25) for measuring the physically detectable change, generating a measurement signal (R) and converting the measurement signal (R) into an intermediate result independent of the evaluation curve by digitization of the measurement signal (R) into a finite number of discrete output states (Xᵢ) and
- a display (7) for indicating the output states in the form of alphanumeric characters, and
c) a readable information carrier element (10a, 12) with a correlation between the alphanumeric codes of the display (7) corresponding to the discrete output states (Xᵢ) and end result values determined in accordance with the evaluation curve.

2. Test carrier analysis system according to claim 1, **characterized in that** the number of output states of the measurement and digitization circuit (25) is less than 40.

3. Test carrier analysis system according to claim 2, **characterized in that** the number of output states of the measurement and digitization circuit (25) is between 10 and 30.

4. Test carrier analysis system according to claim 1, **characterized in that** the measurement of and digitization circuit (25) is designed in such a way that in different parts of the value range of the measurement signal (R) there are assigned varying measurement signal differences (Δ R (Xᵢ)) to an output state (Xᵢ) of the measurement and digitization circuit.

5. Test carrier analysis system according to claim 1, **characterized in that** for the temperature-dependent correction of the intermediate result a temperature sensor (28a) is provided which is linked to the measurement and digitization circuit (25).

## Revendications

1. Système d'analyse de supports de test destiné à analyser un composant d'un échantillon liquide, notamment d'une humeur, comprenant
a) des supports de test qui, dans une ou plusieurs couches de test, contiennent des réactifs, la réaction des réactifs avec le composant produisant une modification physiquement vérifiable d'une couche indicatrice (20), cette modification se trouvant dans une relation déterminée suivant une courbe d'évaluation par rapport à la concentration du composant et la courbe d'évaluation dépendant du lot de fabrication du support de test,
b) un appareil d'exploitation (2) avec
- un circuit de mesure et de numérisation (25) pour la mesure de la modification physiquement vérifiable, la génération d'un signal de mesure (R) et la transformation de ce signal de mesure (R) en un résultat intermédiaire ne dépendant pas de la courbe d'évaluation par numérisation du signal de mesure (R) dans un nombre fini d'états initiaux discrets (Xᵢ) et
- une unité d'affichage (7) pour l'affichage des états initiaux sous forme d'un code alphanumérique
et
c) un élément de support d'informations (10a, 12) avec une assignation entre les codes alphanumériques de l'unité d'affichage (7) correspondant aux états initiaux discrets, et les valeurs des résultats finaux.

2. Système d'analyse de supports de test suivant la revendication 1, **caractérisé** en ce que le nombre des états initiaux du circuit de mesure et de numérisation (25) est inférieur à 40.

3. Système d'analyse de supports de test suivant la revendication 2, **caractérisé** en ce que le nombre des états initiaux du circuit de mesure et de numérisation (25) est compris entre 10 et 30.

4. Système d'analyse de supports de test suivant la revendication 1, **caractérisé** en ce que le circuit de mesure et de numérisation (25) est réalisé de sorte que, dans de différentes parties de la plage des valeurs du signal de mesure (R), de différentes différences de signal de mesure (Δ R (Xᵢ)) sont assignées à un état initial (Xᵢ) du circuit de mesure et de numérisation.

5. Système d'analyse de supports de test suivant la revendication 1, **caractérisé** en ce qu'un capteur de température (28a) est prévu pour la correction du résultat intermédiaire en fonction de la température lequel est relié au circuit de mesure et de numérisation (25).
